# EUROPEAN PATENT APPLICATION

(11) **EP 0 773 029 A1**
(43) Date of publication of application: **14.05.1997**
(21) Application number: 94921121.3
(22) Date of filing: 19.07.1994
(51) Int. Cl.: A61K 31/575, C07J 9/00

(54) **HEPATITIS C VIRUS PROLIFERATION INHIBITOR**

(30) Priority: 19.07.1993 JP 178072/93; 17.11.1993 JP 288391/93
(71) Applicant: TOKYO TANABE COMPANY LIMITED, Chuo-ku Tokyo 103 (JP)
(72) Inventor: OZEKI, Tsuneo, Kitakyushu-shi Fukuoka 807 (JP)
(74) Representative: Matthews, Derek Peter
(86) International application number: PCT/JP94/01187
(87) International publication number: WO 95/03056

(57) **Abstract**

A hepatitis C virus proliferation inhibitor containing bile acid or a physiologically acceptable salt thereof as the active ingredient. Examples of the like acid usable herein include free bile acids such as ursodeoxycholic and chenodeoxycholic acids and conjugated bile acids such as tauroursodeoxycholic acid. Examples of the salt include alkali metal salts such as sodium salts. These acids remarkably inhibit hepatitis C virus proliferation and have extremely high safety, thus being useful as a hepatitis C virus proliferation inhibitor.

## Description

### FIELD OF THE INVENTION

The present invention relates to a hepatitis C virus proliferation inhibitor comprising bile acid as the active principle.

### BACKGROUND ART

Non-A, non-B hepatitis forms 95 to 100% of post-transfusion hepatitis and 40 to 50% of sporadic hepatitis. It may easily become chronic, leading to high rates of cancer of the liver as a result of chronic hepatitis or hepaticcirrhosis. Recently, hepatitis C virus (hereinafter referred to as HCV) was identified, and it has been demonstrated that most cases of hepatitis previously described as non-A or non-B hepatitis are caused by the hepatitis C virus.

Although interferon is known to be an agent which has an inhibitory effect on the proliferation of hepatitis C virus, it is noted that there are problems with it use, such as its low rate of effectiveness (as little as 30 to 40%), 60 to 70% recrudescence after discontinuing use, the appearance of influenza-like symptoms (such as pyrexia, headache and vomiting) and of diverse side effects such as leukopaenia, at high rates.

On the other hand, bile acid is a drug well known as a choleretic, liver function improving agent, etc. Examples of the inhibitory effect of bile acid on viral proliferation, for example, its anti-viral activity against herpesvirus, human immunodeficiency virus, influenza virus, parainfluenza virus, etc. have been published (see Japanese Patent Laid-open Nos. Sho 59-167517, Sho 63-301823, Hei 2-167235, Hei 4-500514 and Hei 4-500670). Bile acid has not yet, however, been shown to have an inhibitory effect on the proliferation of hepatitis C virus. Although a suppository preparation comprising an ursodeoxycholic acid salt and interferon was disclosed in Japanese Patent Laid-open No. Sho 62-77333, the bile acid was in this case only used as an absorption promoting agent in the administration of interferon to the colon or rectum. The disclosure does not therefore suggest that bile acid has any inhibitory effect on the proliferation of hepatitis C virus.

One aim of the present invention is to provide a novel and highly effective hepatitis C virus proliferation inhibitor which gives no adverse reaction.

### DISCLOSURE OF THE INVENTION

As a consequence of the extensive investigation made by the inventors of the present invention in respect of hepatitis C and hepatitis C virus, it has been shown that bile acid exhibits an inhibitory effect on the proliferation of hepatitis C virus. The present invention is directed to a hepatitis C virus proliferation inhibitor comprising bile acid or a physiologically acceptable salt thereof as the active principle.

Examples of bile acids which may be used in the present invention, are free bile acid, such as ursodeoxycholic acid and chenodeoxycholic acid, or conjugated bile acid such as tauroursodeoxycholic acid. Examples of physiologically acceptable salts of bile acids which may be used are alkali metal salts thereof such as the sodium salt. These salts can be prepared according to any of the methods of manufacturing bile acid which are widely known in the art.

Examples of the means for administration of the hepatitis C virus proliferation inhibitor according to the present invention are oral administration as tablets, capsules, granules, powders, medicated syrup, etc, parenteral administration with injections, or suppositories, etc. These pharmaceutical preparations can be prepared by combining the active agent with additives, such as fillers, binders, disintegrators, lubricants, stabilizers and correctives according to any method widely known in the art. The daily adult dose range of the inhibitory agent, judged according to symptoms, age of the patients and other factors, is normally from 800 to 3,000 mg, preferably from 1,200 to 1,800 mg, and more preferably from 1,400 to 1,600 mg for oral administration, and from 30 to 1,200 mg, preferably from 50 to 1,000 mg, and more preferably from 100 to 600 mg for parenteral administration. It may be delivered as a single dose or divided into a number of doses and administered at different times throughout the day. In the case of the parenteral administration, it is preferable to administer the agent by intravenous drip infusion.

### BEST MODE FOR CARRYING OUT THE INVENTION

The results obtained from experiments testing the inhibitory effects of bile acids on the proliferation of hepatitis C virus, clinical tests and acute toxicological tests with bile acid are described hereinafter in detail. Ursodeoxycholic acid (UDCA) and tauroursodeoxycholic acid (TUDCA) were used as representative bile acids.

### Inhibition Test on the Proliferation of Hepatitis C Virus

A liver biopsy was conducted on six patients aged between 40 and 60 suffering from chronic hepatitis C and testing positive for both HCV antibody and HCV RNA. The patients are referred to as S₁, S₂, S₃, S₄, S₅ and S₆, respectively. Half of each liver specimen obtained from the patients, was fixed with formalin and subsequently stained with HE and Azan in order to perform tissue diagnosis. Each of the other halves was ground down in a mortar with sea sand to obtain the supernatant. The supernatant was added to Eagle-MEM culture medium (with added 10% FCS) containing cultured Chang cells, then placed in an atmosphere of 95% air containing 5% CO₂ and maintained at 37°C to facilitate infection. The medium was then centrifuged at 1,800 rpm, and the precipitated cells were added to Eagle-MEM culture medium (with previously added FCS and antibiotic) along with UDCA (to a concentration in the medium of: 25 or 50 µM) or TUDCA (to a concentration in the medium of: 20 or 50 µM) or without the addition of any bile acids. The cell concentration was then adjusted to 1 x 10⁵ cells/slide. The cells were cultivated on LAB-TEK Chamber slides for one week in the UDCA-comprising medium and for 2 weeks in the TUDCA-comprising medium, in an atmosphere of 95% air containing 5% CO₂ and maintained at 37°C.

The presence of HCV in these cultured Chang cells was confirmed by the appearance of a 178 bp (base pair) band following HCV RNA reverse transcription PCR as described in Lancet Vol. 335, pages 1419-22, 1990. Also, the infection rate of Chang cells by HCV was determined immunologically by staining HCV using core antibody (Okayama's monoclonal antibody) and counting the positive cells under an optical microscope. For the immunological staining, the ABC (Avidin-Biotin Complex) method using HRS (Horse Radish staining) was employed. In addition, the infection of Chang cells by HCV was confirmed separately by in situ hybridization.

The results of the inhibition test on the proliferation of HCV in the presence of UDCA or TUDCA are shown in Tables 1 and 2, respectively. The control samples show the results obtained by following the same experimental procedure but without the inclusion of the supernatant.

**Table 1**

| Result of Inhibition Test on Proliferation of HCV by UDCA (Infection Rate : %) | | | |
|---|---|---|---|
| | Concentration of UDCA | | |
| | 0 µM | 25 µM | 50 µM |
| Control | 29.7 ± 5.5 | 21.4 ± 0.9 | 22.2 ± 4.1 |
| Patient S₁ | 45.2 ± 4.2 | 35.6 ± 8.1 | 16.6 ± 2.0 |
| Patient S₂ | 50.1 ± 7.4 | 23.9 ± 7.0 | 17.2 ± 3.3 |
| Patient S₃ | 38.9 ± 8.2 | 27.0 ± 6.2 | 18.5 ± 2.8 |

**Table 2**

| Result of Inhibition Test on Proliferation of HCV by TUDCA (Infection Rate: %) | | | |
|---|---|---|---|
| | Concentration of TUDCA | | |
| | 0 µM | 20 µM | 50 µM |
| Control | 8.4 ± 3.6 | 5.5 ± 5.0 | 2.8 ± 0.6 |
| Patient S₄ | 38.8 ± 7.1 | 32.8 ± 22.4 | 13.5 ± 8.1 |
| Patient S₅ | 18.2 ± 3.3 | 6.9 ± 3.4 | 6.8 ± 0.7 |
| Patient S₆ | 25.6 ± 7.3 | 9.3 ± 3.8 | 5.5 ± 1.2 |

As shown clearly in Tables 1 and 2 above, HCV proliferation was significantly inhibited in both groups with added UDCA or TUDCA, in comparison with the control (no addition of bile acid) group.

### Clinical Test

UDCA was orally administered to each of 8 patients aged from 52 to 72 suffering chronic hepatitis C and referred to as S₇, S₈, S₉, S₁₀, S₁₁, S₁₂, S₁₃ and S₁₄, respectively, at a dose of 1,200 to 1,500 mg/day for a period of 0.5 to 6 months. The concentration of HCV RNA present in the serum of each of the patients both before and after the administration of UDCA are shown in Table 3.

**Table 3**

| Concentration of HCV RNA in Serum both before and after Dosage with UDCA | | | | |
|---|---|---|---|---|
| | Dose (mg/day) | Dosage Period (Month | Concentration of HCV RNA in Serum | |
| | | | Before Dosage | After Dosage |
| Patient S₇ (Female, Age 58) | 1500 | 1 | 10⁹ /ml | 10⁷ /ml |
| Patient S₈ (Male, Age 65) | 1500 | 1 | 10³ /ml | 0 /ml |
| Patient S₉ (Male, Age 59) | 1200-1500 | 6 | 10¹⁰ /ml | 10^{6.5} /ml |
| Patient S₁₀ (Female, Age 72) | 1200-1500 | 5 | 10¹⁰ /ml | 10⁶ /ml |
| Patient S₁₁ (Female, Age 60) | 1500 | 1.5 | 10¹⁰ /ml | 10⁶ /ml |
| Patient S₁₂ (Female, Age 67) | 1500 | 2 | 10¹⁰ /ml | 10^{6.5} /ml |
| Patient S₁₃ (Male, Age 57) | 1500 | 0.5 | 10⁴ /ml | 0 /ml |
| Patient S₁₄ (Male, Age 52) | 1200-1500 | 3 | 10⁴ /ml | 0 /ml |

As can be seen from the Table 3 above, HCV proliferation in patients suffering chronic hepatitis C was remarkably inhibited by the administration of UDCA.

### Acute Toxicological Test

Acute toxicological tests were conducted using male and female Wistar strain rats aged 9 weeks and male and female dd-strain mice aged 8 weeks for UDCA, and by using male and female Beagle dogs aged from 8 to 11 months and male and female SD-strain rats aged 6 weeks for TUDCA to obtain LD₅₀ values for each bile acid respectively. The results are shown in Tables 4 and 5.

**Table 4**

| Results of Acute Toxicological Test for UDCA [LD₅₀ (mg/Kg)] | | | | | |
|---|---|---|---|---|---|
| | | Rats | | Mice | |
| | | Male | Female | Male | Female |
| UDCA | Oral | > 5000 | > 5000 | > 10000 | > 10000 |
| | Intravenous | 310 | 320 | 285 | 240 |

**Table 5**

| Result of Acute Toxicological Test for TUDCA [LD₅₀ (mg/Kg)] | | | | | |
|---|---|---|---|---|---|
| | | Beagle Dogs | | Rats | |
| | | Male | Female | Male | Female |
| TUDCA | Oral | > 5000 | > 5000 | > 10000 | > 10000 |
| | Intravenous | 300-600 | 300-600 | 600-800 | 600-800 |

As can be seen from the Tables 4 and 5 above, the acute toxicity results for both UDCA and TUDCA are very low showing that these bile acids can be safe drugs.

### UTILIZATION IN INDUSTRY

It has been demonstrated that bile acids can inhibit the proliferation of hepatitis C virus remarkably. It has a low level of toxicity, and therefore, may be used as a hepatitis C virus proliferation inhibitor.

## Claims

1. A hepatitis C virus proliferation inhibitor comprising ursodeoxycholic acid, tauroursodeoxycholic acid or a physiologically acceptable salt thereof as the active principle.

2. Use of ursodeoxycholic acid, tauroursodeoxycholic acid or a physiologically acceptable salt thereof in the manufacture of a hepatitis C virus proliferation inhibitor for the treatment of Hepatitis C infection.
